# EUROPÄISCHE PATENTANMELDUNG

(11) **EP 1 464 288 A1**
(43) Veröffentlichungstag der Anmeldung: **06.10.2004**
(21) Anmeldenummer: 04007556.6
(22) Anmeldetag: 29.03.2004
(51) Int. Cl.: A61B 17/22

(54) **Ultraschallanordnung**

(30) Priorität: 02.04.2003 DE 10315055
(71) Anmelder: Dornier MedTech Systems GmbH, 82234 Wessling (DE)
(72) Erfinder: Buchbauer, Peter, 85748 Garching (DE)
(74) Vertreter: Grünecker, Kinkeldey, Stockmair & Schwanhäusser Anwaltssozietät

(57) **Zusammenfassung**

Die vorliegende Erfindung betrifft eine Ultraschallanordnung (1) für medizinische Geräte, insbesondere Lithotripter, mit einer Ultraschalleinheit (3), die von einem Haltearm (4) mit wenigstens zwei gelenkig miteinander verbundenen Armgliedern (6,7) getragen wird, wobei eine Hauptachse (12) der Ultraschalleinheit (3) stets durch ein Isozentrum (10) verläuft. Um eine gattungsgemäße Ultraschallanordnung auf möglichst einfache Weise so zu verbessern, dass eine Ultraschalleinheit möglichst einfach in reproduzierbaren Bahnen verfahrbar ist und die Ultraschallanordnung gleichzeitig stabiler ist, wird ein zweiter Haltearm (5) mit wenigstens zwei gelenkig miteinander verbundenen Armgliedern (13,14) an der Ultraschalleinheit (3) angelenkt und bildet zusammen mit dem ersten Haltearm (4) eine Zwangsführung (25) für die Ultraschalleinheit (3) aus.

## Beschreibung

Die vorliegende Erfindung betrifft eine Ultraschallanordnung für medizinische Geräte, insbesondere Lithotripter, mit den Merkmalen des Oberbegriffes des Anspruches 1.

Aus der DE 199 03 877 C1 ist eine gattungsgemäße Ultraschallanordnung mit einem einseitig bogenförmig auskragenden Haltearm bekannt, an dessen Ende ein Ultraschallwandler gehalten wird. Der bogenförmige Arm kann mehrere bogenförmige Elemente aufweisen, die hintereinander geschaltet gelenkig miteinander verbunden sind. Damit der Ultraschallwandler stets sphärisch innerhalb eines Kugelsegments relativ zur Körperoberfläche bewegt werden kann, sind die Elemente des Arms bogenförmig, kreisförmig gekrümmt. Die Elemente können hintereinander geschaltet drehbar oder teleskopierbar miteinander verbunden sein. Der bogenförmige Arm kann aber auch nur aus einem einzelnen Element bestehen. In jedem Fall ist aber die Anzahl der Gelenkstellen um eins größer als die Anzahl der Elemente des Arms.

Bei dieser Anordnung kann es beim Gebrauch zu Abweichungen des Verlaufs der akustischen Achse der Ultraschalleinheit gegenüber dem Fokus der Therapieeinheit kommen und sie gewährleistet beim Einsatz nicht immer in ausreichender Qualität eine reproduzierbare Bahn.

Der vorliegenden Erfindung liegt die Aufgabe zugrunde, eine gattungsgemäße Ultraschallanordnung auf möglichst einfache Weise so zu verbessern, dass die Ultraschalleinheit möglichst einfach in reproduzierbaren Bahnen verfahrbar ist, wobei die Ultraschallanordnung gleichzeitig stabiler ist.

Die Aufgabe wird erfindungsgemäß gelöst mit einer Ultraschallanordnung mit den Merkmalen des Anspruches 1.

Durch das Vorsehen der zwei Haltearme ist es möglich, die Ultraschalleinheit einhändig in einer Fläche im Raum zwangszuführen. Dementsprechend ist die Ultraschalleinheit auf einer reproduzierbaren Bahn führbar. Dies hat für einen Anwender insbesondere den Vorteil, mit der Ultraschalleinheit erfasste Objekte, beispielsweise zu zertrümmernde Konkremente im Körper eines Patienten, stets aus gleichen Perspektiven mit der Ultraschalleinheit betrachten und vergleichen zu können. Darüber hinaus verleihen die beiden Haltearme der Ultraschallanordnung ein hohes Maß an Stabilität, was ein gutes Beibehalten einer Ausrichtung der Hauptachse der Ultraschalleinheit auf das Isozentrum gewährleistet. Damit hat man kein einseitig bogenförmig auskragendes Element, sondern zwei Haltearme, die sich gegenseitig beeinflussend eine Zwangsführung für die Ultraschalleinheit bilden.

In einer besonders bevorzugten Ausführungsform der Erfindung können die Gelenkachsen der Gelenke der Haltearme durch das Isozentrum verlaufen. Hierdurch ist eine gesamte Hemisphäre oberhalb einer Therapieeinrichtung des Lithotripters mit der Ultraschalleinheit erreichbar.

In einer besonders günstigen Ausführungsform der Erfindung können der Ultraschalleinheit gegenüberliegende Enden der Haltearme gelenkig miteinander verbunden sein. Somit bilden die Haltearme ein geschlossenes System mit einer in sich definierten Bewegungsmechanik.

Besonders vorteilhaft können die Haltearme entlang eines Umfanges einer Therapieeinheit gemeinsam verfahrbar gelagert sein. Dies ermöglicht ein Verfahren eines Systems mit Ultraschalleinheit und Haltearmen entlang des Umfanges der Therapieeinheit.

Bei einer Weiterbildung der Erfindung kann wenigstens ein Gelenk der Haltearme etwa in einer Richtung der Hauptachse der Ultraschalleinheit gegenüber dem lsozentrum zurückversetzt angeordnet sein. Damit wird beim Anwenden der Ultraschallanordnung ein größerer Abstand zwischen diesem Gelenk und einem mit der Ultraschalleinheit zu untersuchenden Bereich im Körper eines Patienten gewährleistet.

Günstigerweise kann wenigstens ein zwei Armglieder der Haltearme verbindendes Gelenk eine Zwangsführung aufweisen. Mit dieser Zwangsführung sind wenigstens die zwei Armglieder relativ zueinander kontrolliert führbar.

Besonders vorteilhaft kann wenigstens ein zwei Armglieder der Haltearme verbindendes Gelenk eine Bremsvorrichtung aufweisen. Damit sind wenigstens die zwei Armglieder einander gegenüber bremsbar.

In einer weiteren vorteilhaften Ausführungsform der Erfindung können wenigstens die Abstände von Gelenken einander paarweise entsprechender Armglieder der Haltearme jeweils gleich sein. Dadurch ist die Ultraschalleinheit in einer Ebene führbar.

Vorteilhafterweise kann eines der Armglieder, die an der Ultraschalleinheit angelenkt sind, gegenüber der Ultraschalleinheit drehbeweglich sein. Hiermit ist das Armglied umfänglich zu der Ultraschalleinheit bewegbar.

Besonders bevorzugt können beide Armglieder, die an der Ultraschalleinheit angelenkt sind, gegenüber der Ultraschalleinheit drehbeweglich sein. Dadurch sind beide Armglieder jeweils umfänglich zu der Ultraschalleinheit bewegbar.

Separater Schutz wird beansprucht für ein medizinisches Gerät, insbesondere Lithotripter, mit einer Ultraschallanordnung mit den Merkmalen nach wenigstens einem der Ansprüche 1 bis 10.

Eine Ausführungsform der vorliegenden Erfindung ist in der Zeichnung dargestellt. Es zeigen:
- Figur 1: eine erfindungsgemäße Ultraschallanordnung in einer ersten Position und
- Figur 2: die Ultraschallanordnung in einer zweiten Position.

Nachfolgend werden für gleiche Elemente stets gleiche Bezugszeichen verwendet. Um Wiederholungen zu vermeiden, wird auf jeweils bereits erfolgte oder noch folgende Beschreibungen zu den jeweiligen Elementen verwiesen.

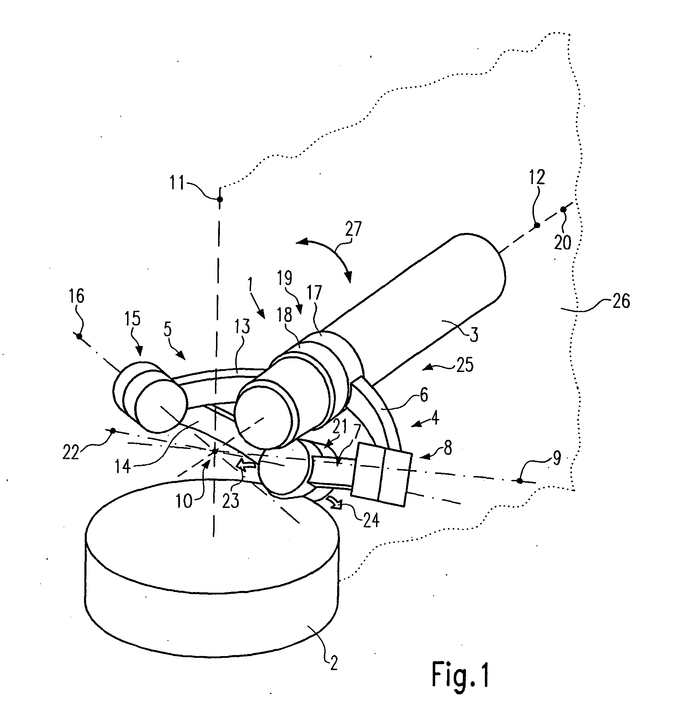

Figur 1 zeigt schematisch eine erfindungsgemäße Ultraschallanordnung 1 und eine Therapieeinheit 2. Die Ultraschallanordnung 1 weist eine Ultraschalleinheit 3, einen ersten Haltearm 4 und einen zweiten Haltearm 5 auf. Beide Haltearmen 4, 5 tragen die Ultraschalleinheit 3 gegenüber der Therapieeinheit 2.

Der erste Haltearm 4 weist ein erstes Armglied 6 und ein zweites Armglied 7 auf. Das erste und zweite Armglied 6, 7 sind mit Hilfe eines ersten Gelenkes 8 um eine erste Gelenkachse 9 schwenkbar gelenkig miteinander verbunden. Die erste Gelenkachse 9 verläuft durch einen Fokus 10 der Therapieeinheit 2, der auf einer Hauptachse 11 der Therapieeinheit 2 liegt.

Durch den Fokus 10 verläuft auch eine Hauptachse 12 der Ultraschalleinheit 3. Der Fokus 10 bildet ein Isozentrum, um das die Ultraschalleinheit 3 mit Hilfe des ersten und zweiten Haltearmes 4, 5 führbar ist. Dabei bleibt der Verlauf der Hauptachse 12 der Ultraschalleinheit 3 durch den das Isozentrum darstellenden Fokus 10 stets erhalten.

Der zweite Haltearm 5 weist ein drittes Armglied 13 und ein viertes Armglied 14 auf. Das dritte und vierte Armglied 13, 14 sind mit Hilfe eines zweiten Gelenkes 15 um eine zweite Gelenkachse 16 schwenkbar gelenkig miteinander verbunden. Auch die zweite Gelenkachse 16 verläuft durch den Fokus 10.

Das erste und dritte Armglied 4, 13 weisen jeweils ein ringförmiges Gelenkelement 17, 18 auf. Mit Hilfe der ringförmigen Gelenkelemente 17, 18 sind das erste und dritte Armglied jeweils drehbeweglich an der Ultraschalleinheit 3 angelenkt. Beide ringförmigen Gelenkelemente 17, 18 bilden ein drittes Gelenk 19 aus. Mittels des dritten Gelenkelementes 19 sind das erste und dritte Armglied 6, 13 um eine dritte Gelenkachse 20 schwenkbar gelenkig miteinander verbunden. In dieser Ausführungsform der Erfindung fällt die dritte Gelenkachse 20 mit der Hauptachse 12 der Ultraschalleinheit 3 zusammen und verläuft durch den Fokus 10.

Das zweite und das vierte Armglied 7, 14 sind über ein viertes Gelenk 21 gelenkig miteinander verbunden. Das vierte Gelenk 21 ermöglicht ein Schwenken des zweiten und vierten Armgliedes 7, 14 um eine vierte Gelenkachse 22, die ebenfalls durch den Fokus 10 verläuft. Somit sind der Ultraschalleinheit 3 gegenüberliegende Enden der Haltearme 4, 5 gelenkig miteinander verbunden.

Das erste und dritte Armglied 6, 13 sowie das zweite und vierte Armglied 7, 14 sind einander jeweils paarweise entsprechende Armglieder der Haltearme 4, 5. Der Abstand des ersten Gelenkes 8 zum dritten Gelenk 19 sowie der Abstand des zweiten Gelenkes 15 zum dritten Gelenk 19 ist jeweils gleich. Auch der Abstand vom ersten Gelenk 8 zum vierten Gelenk 21 sowie der Abstand vom zweiten Gelenk 15 zum vierten Gelenk 21 ist jeweils gleich. Hierdurch ist die Ultraschalleinheit 3 in Ebenen im Raum führbar. In Figur 1 ist eine Ebene 26 dargestellt, in der die Ultraschalleinheit in Pfeilrichtung 27 schwenkbar ist. In der Ebene 26 verläuft die Hauptachse 11 der Therapieeinrichtung 2.

Die ersten bis vierten Armglieder 6, 7, 13,14 sind jeweils etwa rund ausgebildet.

In einer anderen Ausführungsform der Erfindung können eines oder mehrere der Armglieder von einer runden Form abweichend ausgebildet sein. Außerdem kann wenigstens eines der Gelenke etwa in einer Richtung der Hauptachse 12 der Ultraschalleinheit 3 gegenüber dem das Isozentrum darstellenden Fokus 10 zurückversetzt angeordnet sein.

Das vierte Gelenk 21 weist eine Zwangsführung auf. Durch diese Zwangsführung vollziehen das zweite und das vierte Armglied 7, 14 voneinander abhängige Bewegungen. In dieser Ausführungsform der Erfindung sind diese Bewegungen spiegelbildlich zueinander. Gemäß der Zwangsführung des vierten Gelenkes 21 werden auch das erste und dritte Armglied 6, 13 sowie die Ultraschalleinheit 3 zwangsgeführt.

Ferner weist das vierte Gelenk 21 eine Bremsvorrichtung auf. Mit Hilfe der Bremsvorrichtung sind das zweite und vierte Armglied 7, 14 einander gegenüber bremsbar und arretierbar. Durch ein Bremen oder Arretieren des zweiten und vierten Armgliedes 7, 14 werden automatisch auch das erste und dritte Armglied 6, 13 sowie die Ultraschalleinheit 3 gebremst oder fixiert.

Die Haltearme 4, 5 sind über das vierte Gelenk 21 in den Pfeilrichtungen 23, 24 entlang eines Umfanges der Therapieeinheit 2 an der Therapieeinheit 2 verfahrbar gelagert. Hierdurch ist die gesamte Ultraschallanordnung 1 in Umfangsrichtung der Therapieeinrichtung 2 verfahrbar, und zwar unabhängig von der Stellung der Ultraschallanordnung 1.

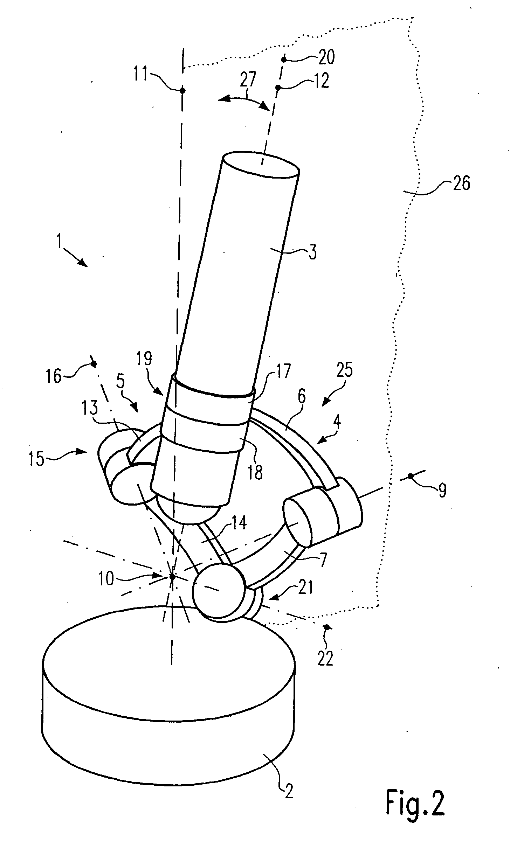

In Figur 1 ist die Ultraschallanordnung 1 in einer ersten Stellung gezeigt. Figur 2 zeigt die Ultraschallanordnung 1 in einer zweiten Stellung, durch die die Ultraschalleinheit 3 durch Schwenken in Pfeilrichtung 27 in der Ebene 26 gelagert ist.

Ein medizinisches Gerät, insbesondere ein Lithotripter, kann wenigstens eine erfindungsgemäße Ultraschallanordnung 1 aufweisen.

Nachfolgend wird die Funktion der erfindungsgemäßen Ultraschallanordnung 1 näher beschrieben.

Beide an die Ultraschalleinheit 3 angelenkten Haltearme 4, 5 bilden eine scherenartige Zwangsführung 25 der Ultraschalleinheit 3. Die scherenartige Zwangsführung 25 ermöglicht das Zwangsführen der Ultraschalleinheit 3 in einer Raumfläche. Weil die Abstände der Gelenke einander paarweise entsprechende Armglieder jeweils gleich sind, ist die Ultraschalleinheit 3 gemäß dieser Ausführungsform der Erfindung in der Ebene 26 führbar, in der auch die Hauptachse 11 der Therapieeinheit 2 liegt. Beim Führen der Ultraschalleinheit 3 in Pfeilrichtung 27 bewegt sich die Hauptachse 12 der Ultraschalleinheit 3 innerhalb der Ebene 26. Auch während des Bewegens der Ultraschalleinheit 3 verlaufen die erste bis vierte Gelenkachse 9, 16, 20, 24 und entsprechend die Hauptachse 12 der Ultraschalleinheit 3 stets durch den das Isozentrum darstellenden Fokus 10. Beim Bewegen der Ultraschallanordnung 1 entlang des Umfanges der Therapieeinrichtung 2 wird die Ebene 26 mitbewegt.

Das Zwangsführen der Ultraschalleinheit 3 in der Ebene 26 stellt ein reproduzierbares Führen der Ultraschalleinheit 3 dar. Ein Anwender kann dadurch ein und dasselbe Objekt stets aus Perspektiven gemäß einer Raumebene untersuchen. Auf diese Weise erhält der Anwender vergleichbare Ansichten.

Das Führen der Ultraschalleinheit 3 kann dabei einhändig und somit sehr bequem durch den Anwender erfolgen. Das Zwangsführen der Ultraschalleinheit 3 erfolgt mit der scherenartigen Zwangsführung 25 dabei automatisch.

## Patentansprüche

1. Ultraschallanordnung (1) für medizinische Geräte, insbesondere Lithotripter, mit einer Ultraschalleinheit (3), die von einem Haltearm (4) mit wenigstens zwei gelenkig miteinander verbundenen Armgliedern (6, 7) getragen wird, wobei eine Hauptachse (12) der Ultraschalleinheit (3) stets durch ein Isozentrum (10) verläuft,
**dadurch gekennzeichnet,**
**dass** ein zweiter Haltearm (5) mit wenigstens zwei gelenkig miteinander verbundenen Armgliedern (13, 14) an der Ultraschalleinheit (3) angelenkt ist und zusammen mit dem ersten Haltearm (4) eine Zwangsführung (25) für die Ultraschalleinheit (3) ausbildet.

2. Ultraschallanordnung nach Anspruch 1,
**dadurch gekennzeichnet,**
**dass** die Gelenkachsen (9, 16, 20, 22) der Gelenke (8, 15, 19, 21) der Haltearme (6, 7, 13, 14) durch das Isozentrum (10) verlaufen.

3. Ultraschallanordnung nach einem der Ansprüche 1 oder 2,
**dadurch gekennzeichnet,**
**dass** der Ultraschalleinheit (3) gegenüberliegende Enden der Haltearme (4, 5) gelenkig miteinander verbunden sind.

4. Ultraschallanordnung nach wenigstens einem der vorhergehenden Ansprüche,
**dadurch gekennzeichnet,**
**dass** die Haltearme (4, 5) entlang eines Umfanges einer Therapieeinheit (2) gemeinsam verfahrbar gelagert sind.

5. Ultraschallanordnung nach wenigstens einem der vorhergehenden Ansprüche,
**dadurch gekennzeichnet,**
**dass** wenigstens ein Gelenk der Haltearme etwa in einer Richtung der Hauptachse (12) der Ultraschalleinheit (3) gegenüber dem Isozentrum (10) zurückversetzt angeordnet ist.

6. Ultraschallanordnung nach wenigstens einem der vorhergehenden Ansprüche,
**dadurch gekennzeichnet,**
**dass** wenigstens ein zwei Armglieder (7, 14) der Haltearme (4, 5) verbindendes Gelenk (21) eine Zwangsführung aufweist.

7. Ultraschallanordnung nach wenigstens einem der vorhergehenden Ansprüche,
**dadurch gekennzeichnet,**
**dass** wenigstens ein zwei Armglieder (7, 14) der Haltearme (4, 5) verbindendes Gelenk (21) eine Bremsvorrichtung aufweist.

8. Ultraschallanordnung nach wenigstens einem der vorhergehenden Ansprüche,
**dadurch gekennzeichnet,**
**dass** wenigstens die Abstände von Gelenken einander paarweise entsprechender Armglieder (6, 13; 7, 14) der Haltearme (4, 5) jeweils gleich sind.

9. Ultraschallanordnung nach wenigstens einem der vorhergehenden Ansprüche,
**dadurch gekennzeichnet,**
**dass** eines der Armglieder (6), die an der Ultraschalleinheit (3) angelenkt sind, gegenüber der Ultraschalleinheit (3) drehbeweglich ist.

10. Ultraschallanordnung nach wenigstens einem der vorhergehenden Ansprüche,
**dadurch gekennzeichnet,**
**dass** beide der Armglieder (6, 13), die an der Ultraschalleinheit (3) angelenkt sind, gegenüber der Ultraschalleinheit (3) beweglich sind.

11. Medizinisches Gerät, insbesondere Lithotritper, mit wenigstens einer Ultraschallanordnung mit den Merkmalen nach wenigstens einem der vorhergehenden Ansprüche.
